# EUROPEAN PATENT SPECIFICATION

(11) **EP 4 479 013 B1**
(45) Date of publication and mention of the grant of the patent: **21.01.2026**
(21) Application number: 23709019.6
(22) Date of filing: 10.02.2023
(51) Int. Cl.: A61K 8/02, A61K 8/11, A61K 8/73, A61Q 5/02

(54) **PERSONAL CARE POD**
KÖRPERPFLEGEBEHÄLTER
DOSETTE DE SOINS PERSONNELS

(30) Priority: 16.02.2022 US 202263310669 P
(43) Date of publication of application: 25.12.2024
(73) Proprietor: Dow Silicones Corporation, Midland MI 48886-0994 (US)
(72) Inventor: NAD, Saugata, 7180 Seneffe (BE); CREUTZ, Serge, 7180 Seneffe (BE); SIMON, Christel, 7180 Seneffe (BE)
(74) Representative: Beck Greener LLP
(86) International application number: PCT/US2023/012753
(87) International publication number: WO 2023/158591

(56) References cited:
- WO-A1-2021/092214
- US-B2- 11 045 397

## Description

The present invention relates to a personal care pod. In particular, the present invention relates to a personal care pod, comprising: a free standing film, wherein the free standing film comprises 20 to 100 wt%, based on weight of the free standing film, of a crosslinked cellulose ether containing 0.1 to 0.6 wt%, based on weight of the crosslinked cellulose ether, of polyether groups; and a personal care formulation; wherein the free standing film forms a cavity; wherein the personal care formulation is disposed within the cavity; wherein the personal care formulation is in contact with the free standing film; and wherein the free standing film encapsulates the personal care formulation.

Personal care products (e.g., shampoo, body wash, hair conditioner, shaving cream) are typically sold to consumers in a liquid or gel format. These personal care products generally contain active ingredients (e.g., surfactants, conditioners) in combination with a significant amount of water. These personal care products are also typically provided commercially to consumers in containers with enough product for a plurality of applications. While such personal care product formats are suitable for many consumer applications, there remains a need for single use formats (e.g., for use in the travel and hospitality industries) and for multiuse concentrated formats that enable the consumer to form a use concentration personal care formulation through addition of water, particularly formats that enable the personal care active ingredients to be packaged and shipped separately from the majority of the water used to make the personal care formulation.

Personal care products have been provided in small plastic bottles for use in the hospitality/travel industries. These formats; however, have an undesirably high packaging to product ratio that contributes to higher costs and increased waste.

In U.S. Patent No. 11,045,397, Stern et al disclose a hygiene product pod and methods of using same. In particular, Stern et al disclose a hygiene product pod comprising: a water-soluble envelope; and an emulsified liquid hygiene product sealed in the envelope, the hygiene product comprising, based on total weight of the hygiene product; surfactants in an around ranging from about 10 wt% to about 40 wt%; a first polyol in an amount ranging from about 10 wt% to about 30 wt%; wherein the first polyol comprises, based on the total weight of the hygiene product: propylene glycol in an amount ranging from about 10 wt% to about 20 wt%; glycerin in an amount ranging from about 1 wt% to about 10 wt%; optionally dipropylene glycol in an amount less than 6 wt%; and hexylene glycol in an amount less than 5 wt%; a second polyol in an amount ranging from about 10 wt% to about 50 wt%, wherein the second polyol is at least one polyglyceryl fatty acid ester (PGE) selected from the group consisting of polyglyceryl-2 caprate, polyglyceryl-3 caprate, polyglyceryl-3 laurate, and polyglyceryl-6 esters; and liquid amides in an amount ranging from about 5 wt% to about 40 wt%, wherein the hygiene product has a free water content of about 9 wt% or less.

One problem with conventionally packaged pods is that the personal care formulations formed therewith have inadequate viscosity given the difficulty of stably formulating conventional rheology modifiers into the concentrated formulations incorporated into the package. The lack of viscosity in the composition formed from the pods can be negatively viewed by consumers and can detrimentally impede performance where rheology can be required, for example, shampoo and body wash formulations.

Accordingly, there remains a continuing need for personal care pods packaged for preparing a personal care product, wherein the personal care product formed has enhanced viscosity. In particular, there remains a need for free standing film compositions that are capable of imparting thickening benefits to personal care formulations formed by personal care pods made with such free standing film compositions.

The present invention provides a personal care pod, comprising: a free standing film, wherein the free standing film comprises 20 to 100 wt%, based on weight of the free standing film, of a crosslinked cellulose ether containing 0.1 to 0.6 wt%, based on weight of the crosslinked cellulose ether, of polyether groups; and a personal care formulation; wherein the free standing film forms a cavity; wherein the personal care formulation is disposed within the cavity; wherein the personal care formulation is in contact with the free standing film; and wherein the free standing film encapsulates the personal care formulation.

The present invention provides a personal care pod, comprising: a free standing film, wherein the free standing film comprises 20 to 100 wt%, based on weight of the free standing film, of a crosslinked cellulose ether containing 0.1 to 0.6 wt%, based on weight of the crosslinked cellulose ether, of polyether groups; and a personal care formulation; wherein the free standing film has an average thickness of 5 to 200 µm; wherein the free standing film forms a cavity; wherein the personal care formulation is disposed within the cavity; wherein the personal care formulation is in contact with the free standing film; and wherein the free standing film encapsulates the personal care formulation.

The present invention provides a personal care pod, comprising: a free standing film, wherein the free standing film comprises 20 to 100 wt%, based on weight of the free standing film, of a crosslinked cellulose ether containing 0.1 to 0.6 wt%, based on weight of the crosslinked cellulose ether, of polyether groups; and a personal care formulation; wherein the crosslinked cellulose ether is an irreversibly crosslinked cellulose ether; wherein the free standing film forms a cavity; wherein the personal care formulation is disposed within the cavity; wherein the personal care formulation is in contact with the free standing film; and wherein the free standing film encapsulates the personal care formulation.

The present invention provides a personal care pod, comprising: a free standing film, wherein the free standing film comprises 20 to 100 wt%, based on weight of the free standing film, of a crosslinked cellulose ether containing 0.1 to 0.6 wt%, based on weight of the crosslinked cellulose ether, of polyether groups; and a personal care formulation; wherein the crosslinked cellulose ether is an irreversibly crosslinked cellulose ether; wherein the polyether groups in the irreversibly crosslinked cellulose ether are polyoxyalkylene groups having 2 to 100 oxyalkylene groups; wherein the free standing film forms a cavity; wherein the personal care formulation is disposed within the cavity; wherein the personal care formulation is in contact with the free standing film; and wherein the free standing film encapsulates the personal care formulation.

The present invention provides a personal care pod, comprising: (i) a free standing film, wherein the free standing film comprises: 20 to 100 wt%, based on weight of the free standing film, of a crosslinked cellulose ether containing 0.1 to 0.6 wt%, based on weight of the crosslinked cellulose ether, of polyether groups; and at least one of: (a) 5 to 80 wt%, based on weight of the free standing film, of a partially hydrolyzed poly(vinyl acetate); (b) 0.1 to 45 wt%, based on weight of the free standing film, of a crosslinking agent; (c) 0.1 to 45 wt%, based on weight of the free standing film, of a poly(ethylene oxide) having a weight average molecular weight of 20,000 to 2,000,000 Daltons; (d) 0.1 to 35 wt%, based on weight of the free standing film, of a poly(alkylene glycol) having a weight average molecular weight of > 400 to 5,300 Daltons; (e) 0.1 to 45 wt%, based on weight of the free standing film, of a plasticizer; (f) 0.1 to 10 wt%, based on weight of the free standing film, of a poly(isobutylene-co-maleic anhydride) copolymer, wherein the poly(isobutylene-co-maleic anhydride) copolymer is at least partially neutralized; and (g) 0.01 to 1.8 wt%, based on weight of the free standing film, of a poly(vinyl pyrrolidone); and (ii) a personal care formulation; wherein the free standing film forms a cavity; wherein the personal care formulation is disposed within the cavity; wherein the personal care formulation is in contact with the free standing film; and wherein the free standing film encapsulates the personal care formulation.

The present invention provides a personal care pod, comprising: (i) a free standing film, wherein the free standing film comprises: 20 to 95 wt%, based on weight of the free standing film, of the crosslinked cellulose ether containing 0.1 to 0.6 wt%, based on weight of the crosslinked cellulose ether, of polyether groups; and 5 to 80 wt%, based on weight of the free standing film, of the partially hydrolyzed poly(vinyl acetate); and (ii) a personal care formulation; wherein the free standing film forms a cavity; wherein the personal care formulation is disposed within the cavity; wherein the personal care formulation is in contact with the free standing film; and wherein the free standing film encapsulates the personal care formulation.

The present invention provides a method of using a personal care pod of the present invention, wherein the personal care pod is a single use personal care pod, comprising: applying water to the single use personal care pod to dissolve or disperse the free standing film and release the personal care formulation; and applying the personal care formulation to skin or hair.

The present invention provides a method of forming a personal care formulation, comprising: providing a personal care pod of the present invention; wherein the personal care pod is a personal care concentrate pod and wherein the personal care formulation contained in the personal care concentrate pod is a personal care formulation concentrate; providing a container; adding water to the container; adding the personal care concentrate pod to the container; agitating the container contents; allowing the water to dissolve or disperse the free standing film of the personal care concentrate pod; and mixing the water with the personal care formulation concentrate from the personal care concentrate pod to form a use concentration personal care formulation in the container.

### DETAILED DESCRIPTION

We have found a unique free standing film that facilitates significant thickening of end use personal care formulations formed using personal care pods comprising same.

Unless otherwise indicated, ratios, percentages, parts, and the like are by weight.

As used herein, unless otherwise indicated, the phrase "molecular weight" or Mw refers to the weight average molecular weight as measured in a conventional manner with gel permeation chromatography (GPC) and polyacrylic acid standards. GPC techniques are discussed in detail in Modem Size Exclusion Chromatography, W. W. Yau, J. J. Kirkland, D. D. Bly; Wiley-Interscience, 1979, and in A Guide to Materials Characterization and Chemical Analysis, J. P. Sibilia; VCH, 1988, p.81-84. Molecular weights are reported herein in units of Daltons.

The term "DS" as used herein and in the appended claims means the number of alkyl substituted OH groups per anhydroglucose unit in a cellulose ether, as determined by the Zeisel Method.

The term "DS (methyl)" or "DS (M)" as used herein and in the appended claims means the number of methyl substituted OH groups per anhydroglucose unit in a cellulose ether, as determined by the Zeisel Method.

The term "MS" as used herein and in the appended claims means the number of moles of etherification reagent which are bound as ether per mol of anhydroglucose unit as hydroxyalkyl substituents in a cellulose ether, as determined by the Zeisel Method.

The term "MS (hydroxyethyl)" or "MS (HE)" as used herein and in the appended claims means the number of moles of etherification reagent which are bound as ether per mol of anhydroglucose unit as hydroxyethyl substituents in a cellulose ether, as determined by the Zeisel Method.

The term "MS (hydroxypropyl)" or "MS (HP)" as used herein and in the appended claims means the number of moles of etherification reagent which are bound as ether per mol of anhydroglucose unit as hydroxypropyl substituents in a cellulose ether, as determined by the Zeisel Method.

The term "Zeisel Method" refers to the Zeisel cleavage procedure for determination of MS and DS. See G. Bartelmus and R. Ketterer, Zeitschrift fuer Analytische Chemie, Vol. 286 (1977, Springer, Berline, DE), pages 161-190.

Preferably, the personal care pod of the present invention, comprises: (i) a free standing film, wherein the free standing film comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups; and (ii) a personal care formulation; wherein the free standing film forms a cavity; wherein the personal care formulation is disposed within the cavity; wherein the personal care formulation is in contact with the free standing film; and wherein the free standing film encapsulates the personal care formulation.

Preferably, the free standing film used in the personal care pod of the present invention, comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups (preferably, wherein the crosslinked cellulose ether is an irreversibly crosslinked cellulose ether).

Preferably, the free standing film used in the personal care pod of the present invention has an average thickness of 5 to 200 µm (preferably, 20 to 100 µm; more preferably, 35 to 100 µm; most preferably, 40 to 85 µm).

Preferably, the free standing film used in the personal care pod of the present invention, comprise has a disintegration time of less than 90 seconds as determined at 40° C using distilled water according to MSTM 205.

Preferably, the free standing film used in the personal care pod of the present invention is water soluble. The term "water soluble" as used herein in reference to a free standing film of the present invention means that a sample of the free standing film (0.5" x 1.5" x 76 µm) when placed in 20 mL of tap water having a temperature of 5 to 25 °C in a sample vial; left to sit undisturbed for two (2) minutes; then shaken for sixty (60) seconds and then filtered through a 0.025 mm mesh screen, wherein only a slight haze is perceptible and no residue or grit is observed according to the procedure set forth herein in the Examples.

Preferably, the free standing film used in the personal care pod of the present invention is water soluble based on film solubility tests conducted according to MSTM (MonoSol Standard Test Method) 205 in distilled water at 25 °C.

Preferably, the free standing film used in the personal care pod of the present invention, comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups; wherein the crosslinked cellulose ether comprises a base cellulose ether and crosslinks; wherein the crosslinks contain the polyether groups and wherein the base cellulose ether is a mixed cellulose ether containing hydroxyalkyl ether groups and alkyl ether groups. More preferably, the free standing film used in the personal care pod of the present invention, comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups; wherein the crosslinked cellulose ether comprises a base cellulose ether and crosslinks; wherein the crosslinks contain the polyether groups and wherein the base cellulose ether is selected from the group consisting of hydroxyethyl methylcellulose, hydroxypropyl methyl cellulose, methyl hydroxyethyl hydroxypropylcellulose, ethyl hydroxyethyl cellulose and combinations thereof. Most preferably, the free standing film used in the personal care pod of the present invention, comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups; wherein the crosslinked cellulose ether comprises a base cellulose ether and crosslinks; wherein the crosslinks contain the polyether groups and wherein the base cellulose ether is hydroxyethyl methylcellulose.

Preferably, the free standing film used in the personal care pod of the present invention, comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups; wherein the crosslinked cellulose either is an irreversibly crosslinked cellulose ether. More preferably, the free standing film used in the personal care pod of the present invention, comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups; wherein the crosslinked cellulose ether comprises a base cellulose ether and crosslinks; wherein the crosslinks contain the polyether groups; wherein the base cellulose ether is a mixed cellulose ether containing hydroxyalkyl ether groups and alkyl ether groups and wherein the crosslinked cellulose either is an irreversibly crosslinked cellulose ether. Still more preferably, the free standing film used in the personal care pod of the present invention, comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups; wherein the crosslinked cellulose ether comprises a base cellulose ether and crosslinks; wherein the crosslinks contain the polyether groups; wherein the base cellulose ether is selected from the group consisting of hydroxyethyl methylcellulose, hydroxypropyl methyl cellulose, methyl hydroxyethyl hydroxypropylcellulose, ethyl hydroxyethyl cellulose and combinations thereof and wherein the crosslinked cellulose either is an irreversibly crosslinked cellulose ether. Most preferably, the free standing film used in the personal care pod of the present invention, comprises: 20 to 100 wt% (preferably, at least 25 wt%; more preferably, at least 40 wt%; most preferably, at least 60 wt%)(preferably, no more than 98 wt%; more preferably, no more than 95 wt%; most preferably, no more than 90 wt%), based on weight of the free standing film, of a crosslinked cellulose ether containing 0.1 to 0.6 wt%, based on weight of the crosslinked cellulose ether, of polyether groups; wherein the crosslinked cellulose ether comprises a base cellulose ether and crosslinks; wherein the crosslinks contain the polyether groups; wherein the base cellulose ether is hydroxyethyl methylcellulose and wherein the crosslinked cellulose either is an irreversibly crosslinked cellulose ether.

Preferably, the crosslinked cellulose ether contains 0.1 to 0.6 wt% (preferably, 0.12 to 0.6 wt%; more preferably, 0.12 to 0.45 wt%; most preferably, 0.12 to 0.29 wt%), based on weight of the crosslinked cellulose ether, of polyether groups. More preferably, the crosslinked cellulose ether contains 0.1 to 0.6 wt% (preferably, 0.12 to 0.6 wt%; more preferably, 0.12 to 0.45 wt%; most preferably, 0.12 to 0.29 wt%), based on weight of the crosslinked cellulose ether, of polyether groups; wherein the polyether groups are polyoxyalkylene groups having 2 to 100 (preferably, 2 to 20; more preferably, 3 to 15) oxyalkylene groups per crosslink. Most preferably, the crosslinked cellulose ether contains 0.1 to 0.6 wt% (preferably, 0.12 to 0.6 wt%; more preferably, 0.12 to 0.45 wt%; most preferably, 0.12 to 0.29 wt%), based on weight of the crosslinked cellulose ether, of polyether groups; wherein the polyether groups are polyoxypropylene groups having 2 to 100 (preferably, 2 to 20; more preferably, 3 to 15) oxypropylene groups per crosslink.

Preferably, crosslinked cellulose ether comprises a base cellulose ether having crosslinks containing 0.1 to 0.6 wt%, based on weight of the crosslinked cellulose ether, of polyether groups. Preferably, the base cellulose ether is selected from hydroxyalkyl cellulose ethers, alkyl cellulose ethers and combinations thereof. Examples of base cellulose ethers include, for example, methylcellulose, ethylcellulose, propylcellulose, butylcellulose, hydroxyethyl methylcellulose, hydroxypropyl methylcellulose, hydroxyethylcellulose, ethylhydroxyethylcellulose, methylethylhydroxyethylcellulose, hydrophobically modified ethylhydroxyethylcellulose, hydrophobically modified hydroxyethylcellulose, sulfoethyl methylhydroxyethylcellulose, sulfoethyl methylhydroxypropylcellulose and sulfoethyl hydroxyethylcellulose. Preferably, the base cellulose ethers are mixed cellulose ethers that contain both hydroxyalkyl ether groups and alkyl ether groups, such as, alkyl hydroxyethyl cellulose and hydroxyalkyl methylcellulose (e.g., hydroxyethyl methylcellulose, hydroxypropyl methylcellulose, methyl hydroxyethyl hydroxypropylcellulose and ethylhydroxyethyl cellulose).

Preferably, the base cellulose ether contains hydroxyalkyl ether substitutions. More preferably, the base cellulose ether has a degree of hydroxyethyl ether substitutions, MS (HE), or hydroxypropyl ether substitutions, MS (HP), of 1.5 to 4.5 (preferably, 2.0 to 3.0).

Preferably, the base cellulose ether contains methyl ether substitutions. More preferably, the base cellulose ether has a degree of methyl ether substitution, DS (M), of 1.2 to 2.1 (preferably, 1.3 to 1.7; more preferably, 1.35 to 1.60).

Preferably, the base cellulose ether is a mixed cellulose ether containing hydroxyalkyl ether substitutions and alkyl ether substitutions. More preferably, the base cellulose ether is a mixed cellulose ether having a degree of hydroxyethyl ether substitution, MS (HE), of 0.05 to 0.75 (preferably, 0.15 to 0.45; more preferably, 0.20 to 0.40) and a degree of methyl ether substitution, DS (M), of 1.2 to 2.1 (preferably, 1.3 to 1.7, more preferably, 1.35 to 1.60).

Preferably, the base cellulose ether is a mixed cellulose ether containing hydroxyalkyl ether substitutions and alkyl ether substitutions. More preferably, the base cellulose ether is a mixed cellulose ether having a degree of hydroxypropyl ether substitution, MS (HP), of 0.1 to 1.5 (preferably, 0.2 to 1.2) and a degree of methyl ether substitution, DS (M), of 1.2 to 2.1 (preferably, 1.3 to 2.0).

Preferably, the crosslinked cellulose ether comprises a base cellulose ether having crosslinks containing 0.1 to 0.6 wt%, based on weight of the crosslinked cellulose ether, of polyether groups; wherein the base cellulose ether is a hydroxyethyl methyl cellulose and wherein the crosslinks are polyoxypropylene dioxyethylene ether crosslinks, such as those produced as the reaction product of hydroxyethyl methyl cellulose with polypropylene glycol (PPG) glycidylether.

Crosslinking agents used to crosslink the base cellulose ether to form the crosslinked cellulose ether include compounds having a polyoxyalkylene or poly(alkylene glycol) group and two or more (preferably, two) crosslinking groups, such as, halogen groups, glycidyl or epoxy groups, and ethylenically unsaturated groups (e.g., vinyl groups) that form ether bonds with the base cellulose ether to form the crosslinked cellulose ether. Preferably, the crosslinking agent is selected from the group consisting of 1,2-dichloro(poly)alkoxy ethers, dichloropolyoxyethylene, diglycidyl polyalkoxy ethers, diglycidyl phosphonate, divinyl polyoxyalkylenes containing a sulphone group. Crosslinking agents having two different types of functional groups can be used. Examples include diglycidyl polyoxypropylenes and glycidyl(poly)oxyalkyl methacrylate. Preferably, the crosslinking agent contains 2 to 100 (preferably, 2 to 20; more preferably, 3 to 15) oxyalkylene groups per molecule.

Preferably, the amount of crosslinking agent included in the crosslinked cellulose ether ranges from 0.0001 to 0.05 eq (preferably, 0.0005 to 0.01 eq; more preferably, 0.001 to 0.005 eq), wherein the unity "eq" represents the molar ratio of moles of the crosslinking agent relative to the number of moles of anhydroglucose units (AGU) in the base cellulose ether.

Preferably, the crosslinked cellulose ether is an irreversibly crosslinked cellulose ether. That is, the crosslinks in the crosslinked cellulose ether do not break down during the intended use of the crosslinked cellulose ether under normal conditions. In contrast, reversible crosslinks will break down during the intended use of the crosslinked cellulose ether under normal conditions. An example of reversible crosslinks in cellulose ethers intended for use in laundry detergent formulations are those created using aldehyde based crosslinkers (e.g., glyoxal), which crosslinks break down upon dissolution of the crosslinked material in water.

Preferably, the free standing film used in the personal care pod of the present invention, comprises at least one of (a) 5 to 80 wt% (preferably, 5 to 75 wt%; more preferably, 7.5 to 60 wt%; most preferably, 10 to 40 wt%), based on weight of the free standing film, of a partially hydrolyzed poly(vinyl acetate) (preferably, wherein the partially hydrolyzed poly(vinyl acetate) is 75 to 99% hydrolyzed (more preferably, 80 to 99% hydrolyzed; most preferably, 90 to 99% hydrolyzed)); (b) 0.1 to 45 wt% (preferably, 10 to 40 wt%; more preferably, 15 to 40 wt%; most preferably, 20 to 35 wt%), based on weight of the free standing film, of a crosslinking agent; (c) 0.1 to 45 wt% (preferably, 1 to 40 wt%; more preferably, 5 to 25 wt%; most preferably, 10 to 15 wt%), based on weight of the free standing film, of a poly(ethylene oxide) having a weight average molecular weight of 20,000 to 2,000,000 Daltons (preferably, 100,000 to 1,000,000 Daltons; more preferably, 250,000 to 750,000 Daltons; most preferably, 350,000 to 650,000 Daltons); (d) 0.1 to 35 wt% (preferably, 1 to 25 wt%; more preferably, 2 to 15 wt%; most preferably, 4 to 7.5 wt%), based on weight of the free standing film, of a poly(alkylene glycol) having a weight average molecular weight of > 400 to 5,300 Daltons (preferably, 500 to 1,500 Daltons; more preferably, 750 to 1,200 Daltons; most preferably, 800 to 1,000 Daltons); (e) 0.1 to 45 wt% (preferably, 1 to 40 wt%; more preferably, 2 to 35 wt%; most preferably, 5 to 25 wt%), based on weight of the free standing film, of a plasticizer; (f) 0.1 to 10 wt% (preferably, 0.5 to 7.5 wt%; more preferably, 1 to 6 wt%; most preferably, 2 to 5.5 wt%), based on weight of the free standing film, of a poly(isobutylene-co-maleic anhydride) copolymer, wherein the poly(isobutylene-co-maleic anhydride) copolymer is at least partially neutralized (preferably, wherein the degree of neutralization is 50 to 100%; more preferably, 75 to 100%; most preferably, 95 to 100%); and (g) 0.01 to 1.8 wt%, based on weight of the free standing film, of a poly(vinyl pyrrolidone) (preferably, wherein the poly(vinyl pyrrolidone) has a weight average molecular weight of 5,000 to 2,000,000 Daltons (preferably, 10,000 to 1,500,000 Daltons; more preferably, 20,000 to 100,000 Daltons; most preferably, 20,000 to 50,000 Daltons)(preferably, wherein the weight ratio of the partially hydrolyzed poly(vinyl acetate) to the poly(vinyl pyrrolidone) in the free standing film is > 10:1 (preferably, at least 100:1; more preferably, at least 150:1; most preferably, 150:1 to 600:1).

Preferably, the free standing film used in the personal care pod of the present invention, comprises: 20 to 95 wt% (preferably, at least 25 wt%; more preferably, at least 40 wt%; most preferably, at least 60 wt%)(preferably, no more than 90 wt%), based on weight of the free standing film, of a crosslinked cellulose ether containing 0.1 to 0.6 wt%, based on weight of the crosslinked cellulose ether, of polyether groups; and 5 to 80 wt% (preferably, at least 5 wt%; more preferably, at least 10 wt%)(preferably, no more than 75 wt%; more preferably, no more than 60 wt%; most preferably, no more than 40 wt%), based on weight of the free standing film, of a partially hydrolyzed poly(vinyl acetate) (preferably, 75 to 99% hydrolyzed; more preferably, 80 to 99% hydrolyzed; most preferably, 90 to 99% hydrolyzed).

Preferably, the free standing film used in the personal care pod of the present invention, further comprises: 5 to 80 wt% (preferably, 5 to 75 wt%; more preferably, 7.5 to 60 wt%; most preferably, 10 to 40 wt%)(preferably, at least 5 wt%; more preferably, at least 10 wt%)(preferably, no more than 75 wt%; more preferably, no more than 60 wt%; most preferably, no more than 40 wt%), based on weight of the free standing film, of a partially hydrolyzed poly(vinyl acetate)(preferably, wherein the partially hydrolyzed poly(vinyl acetate) is 75 to 99% hydrolyzed (more preferably, 80 to 99% hydrolyzed; most preferably, 90 to 99% hydrolyzed)). More preferably, the free standing film used in the personal care pod of the present invention, further comprises: 5 to 80 wt% (preferably, 5 to 75 wt%; more preferably, 7.5 to 60 wt%; most preferably, 10 to 40 wt%)(preferably, at least 5 wt%; more preferably, at least 10 wt%)(preferably, no more than 75 wt%; more preferably, no more than 60 wt%; most preferably, no more than 40 wt%), based on weight of the free standing film, of a partially hydrolyzed poly(vinyl acetate); wherein the partially hydrolyzed poly(vinyl acetate) has a weight average molecular weight of 10,000 to 250,000 Daltons (preferably, 20,000 to 175,000 Daltons; more preferably, 30,000 to 100,000 Daltons; most preferably, 55,000 to 80,000 Daltons)(preferably, wherein the partially hydrolyzed poly(vinyl acetate) is 75 to 99% hydrolyzed (more preferably, 80 to 99% hydrolyzed; most preferably, 90 to 99% hydrolyzed)).

Preferably, the free standing film used in the personal care pod of the present invention, further comprises 0.1 to 45 wt% (preferably, 10 to 40 wt%; more preferably, 15 to 40 wt%; most preferably, 20 to 35 wt%), based on weight of the free standing film, of a crosslinking agent. More preferably, the free standing film of the present invention, further comprises 0.1 to 45 wt% (preferably, 10 to 40 wt%; more preferably, 15 to 40 wt%; most preferably, 20 to 35 wt%), based on weight of the free standing film, of a crosslinking agent; wherein the crosslinking agent is an ionic crosslinking agent (e.g., alkali metal salts, alkaline metal salts and mixtures thereof). Preferably, the crosslinking agent is selected from ionic crosslinking agents including Ca²⁺, Mg²⁺, Al²⁺, Al³⁺, Zn²⁺ and mixtures thereof. More preferably, the crosslinking agent is selected from ionic crosslinking agents including Ca²⁺, Zn²⁺ and mixtures thereof. Preferably, the cations are provided as a water soluble inorganic salt or complex, for example, CaCl₂, ZnO, Zinc ammonium bicarbonate.

Preferably, the free standing film used in the personal care pod of the present invention, further comprises 0.1 to 45 wt% (preferably, 1 to 40 wt%; more preferably, 5 to 25 wt%; most preferably, 10 to 15 wt%), based on weight of the free standing film, of a poly(ethylene oxide). More preferably, the free standing film used in the personal care pod of the present invention, comprises 0.1 to 45 wt% (preferably, 1 to 40 wt%; more preferably, 5 to 25 wt%; most preferably, 10 to 15 wt%), based on weight of the free standing film, of a poly(ethylene oxide); wherein the poly(ethylene oxide) has a weight average molecular weight of 20,000 to 2,000,000 Daltons (preferably, 100,000 to 1,000,000 Daltons; more preferably, 250,000 to 750,000 Daltons; most preferably, 350,000 to 650,000 Daltons).

Preferably, the free standing film used in the personal care pod of the present invention further comprises 0.1 to 35 wt% (preferably, 1 to 25 wt%; more preferably, 2 to 15 wt%; most preferably, 4 to 7.5 wt%), based on weight of the free standing film, of a poly(alkylene glycol). More preferably, the free standing film used in the personal care pod of the present invention further comprises 0.1 to 35 wt% (preferably, 1 to 25 wt%; more preferably, 2 to 15 wt%; most preferably, 4 to 7.5 wt%), based on weight of the free standing film, of a poly(alkylene glycol); wherein the poly(alkylene glycol) has a weight average molecular weight of > 400 to 5,300 Daltons (preferably, 500 to 1,500 Daltons; more preferably, 750 to 1,200 Daltons; most preferably, 800 to 1,000 Daltons). Most preferably, the free standing film used in the personal care pod of the present invention further comprises 0.1 to 35 wt% (preferably, 1 to 25 wt%; more preferably, 2 to 15 wt%; most preferably, 4 to 7.5 wt%), based on weight of the free standing film, of a poly(alkylene glycol); wherein the poly(alkylene glycol) has a weight average molecular weight of > 400 to 5,300 Daltons (preferably, 500 to 1,500 Daltons; more preferably, 750 to 1,200 Daltons; most preferably, 800 to 1,000 Daltons) and wherein the poly(alkylene glycol) is a random copolymer of ethylene oxide and propylene oxide.

Preferably, the free standing film used in the personal care pod of the present invention, further comprises 0.1 to 45 wt% (preferably, 1 to 40 wt%; more preferably, 2 to 35 wt%; most preferably, 5 to 25 wt%), based on weight of the free standing film, of a plasticizer. More preferably, the free standing film used in the personal care pod of the present invention, further comprises 0.1 to 45 wt% (preferably, 1 to 40 wt%; more preferably, 2 to 35 wt%; most preferably, 5 to 25 wt%), based on weight of the free standing film, of a plasticizer; wherein the plasticizer is selected from the group consisting of dipropylene glycol, isomalt, maltitol, sorbitol, xylitol, erythritol, adonitol, dulcitol, pentaerythritol, mannitol, glycerin, diglycerin, ethylene glycol, diethylene glycol, triethyleneglycol, tetraethylene glycol, polyethylene glycol having a weight average molecular weight of up to 400 Daltons, neopentyl glycol, propylene glycol, 2-methyl-1,3-propanediol, trimethylolpropane, polyether polyols having a weight average molecular weight of up to 400 Daltons, and mixtures thereof. Still more preferably, the free standing film used in the personal care pod of the present invention, further comprises 0.1 to 45 wt% (preferably, 1 to 40 wt%; more preferably, 2 to 35 wt%; most preferably, 5 to 25 wt%), based on weight of the free standing film, of a plasticizer; wherein the plasticizer is selected from the group consisting of dipropylene glycol, sorbitol, glycerin and mixtures thereof. Most preferably, the free standing film used in the personal care pod of the present invention, further comprises 0.1 to 45 wt% (preferably, 1 to 40 wt%; more preferably, 2 to 35 wt%; most preferably, 5 to 25 wt%) , based on weight of the free standing film, of a plasticizer; wherein the plasticizer includes a mixture of dipropylene glycol, sorbitol and glycerin.

Preferably, the free standing film used in the personal care pod of the present invention, further comprises 0.1 to 10 wt% (preferably, 0.5 to 7.5 wt%; more preferably, 1 to 6 wt%; most preferably, 2 to 5.5 wt%), based on weight of the free standing film, of a poly(isobutylene-co-maleic anhydride) copolymer. More preferably, the free standing film used in the personal care pod of the present invention, further comprises 0.1 to 10 wt% (preferably, 0.5 to 7.5 wt%; more preferably, 1 to 6 wt%; most preferably, 2 to 5.5 wt%), based on weight of the free standing film, of a poly(isobutylene-co-maleic anhydride) copolymer; wherein the poly(isobutylene-co-maleic anhydride) copolymer is at least partially neutralized (preferably, wherein the degree of neutralization is 50 to 100%; more preferably, 75 to 100%; most preferably, 95 to 100%). Most preferably, the free standing film used in the personal care pod of the present invention, further comprises 0.1 to 10 wt% (preferably, 0.5 to 7.5 wt%; more preferably, 1 to 6 wt%; most preferably, 2 to 5.5 wt%), based on weight of the free standing film, of a poly(isobutylene-co-maleic anhydride) copolymer; wherein the poly(isobutylene-co-maleic anhydride) copolymer is at least partially neutralized (preferably, wherein the degree of neutralization is 50 to 100%; more preferably, 75 to 100%; most preferably, 95 to 100%) and wherein the poly(isobutylene-co-maleic anhydride) copolymer has a weight average molecular weight of 50,000 to 500,000 Daltons (preferably, 75,000 to 250,000 Daltons; more preferably, 100,000 to 200,000 Daltons; most preferably, 140,000 to 180,000 Daltons).

Preferably, the free standing film used in the personal care pod of the present invention, further comprises 0.1 to 10 wt% (preferably, 0.5 to 7.5 wt%; more preferably, 1 to 6 wt%; most preferably, 2 to 5.5 wt%), based on weight of the free standing film, of a poly(isobutylene-co-maleic anhydride) copolymer; wherein the poly(isobutylene-co-maleic anhydride) copolymer is at least partially neutralized with at least one of an alkali earth metal hydroxide, an alkaline earth metal hydroxide and an ionomer, wherein the degree of neutralization is 50 to 100% (more preferably, 75 to 100%; most preferably, 95 to 100%). More preferably, the free standing film used in the personal care pod of the present invention, further comprises 0.1 to 10 wt% (preferably, 0.5 to 7.5 wt%; more preferably, 1 to 6 wt%; most preferably, 2 to 5.5 wt%), based on weight of the free standing film, of a poly(isobutylene-co-maleic anhydride) copolymer; wherein the poly(isobutylene-co-maleic anhydride) copolymer is at least partially neutralized with at least one of an alkali earth metal hydroxide and an alkaline earth metal hydroxide, wherein the degree of neutralization is 50 to 100% (more preferably, 75 to 100%; most preferably, 95 to 100%). Most preferably, the free standing film used in the personal care pod of the present invention, further comprises 0.1 to 10 wt% (preferably, 0.5 to 7.5 wt%; more preferably, 1 to 6 wt%; most preferably, 2 to 5.5 wt%) , based on weight of the free standing film, of a poly(isobutylene-co-maleic anhydride) copolymer; wherein the poly(isobutylene-co-maleic anhydride) copolymer is at least partially neutralized with at least one of sodium hydroxide, potasium hydroxide, magnesium hydroxide and calcium hydroxide, wherein the degree of neutralization is 50 to 100% (more preferably, 75 to 100%; most preferably, 95 to 100%).

Preferably, the free standing film used in the personal care pod of the present invention, further comprises 0.01 to 1.8 wt%, based on weight of the free standing film, of a poly(vinyl pyrrolidone). More preferably, the free standing film used in the personal care pod of the present invention, further comprises 0.01 to 1.8 wt%, based on weight of the free standing film, of a poly(vinyl pyrrolidone); wherein the poly(vinyl pyrrolidone) has a weight average molecular weight of 5,000 to 2,000,000 Daltons (preferably, 10,000 to 1,500,000 Daltons; more preferably, 20,000 to 100,000 Daltons; most preferably, 20,000 to 50,000 Daltons). Most preferably, the free standing film used in the personal care pod of the present invention, further comprises 0.01 to 1.8 wt%, based on weight of the free standing film, of a poly(vinyl pyrrolidone); wherein the poly(vinyl pyrrolidone) has a weight average molecular weight of 5,000 to 2,000,000 Daltons (preferably, 10,000 to 1,500,000 Daltons; more preferably, 20,000 to 100,000 Daltons; most preferably, 20,000 to 50,000 Daltons) and wherein the weight ratio of the partially hydrolyzed poly(vinyl acetate) to the poly(vinyl pyrrolidone) in the free standing film is > 10:1 (preferably, at least 100:1; more preferably, at least 150:1; most preferably, 150:1 to 600:1).

Preferably, the free standing film used in the personal care pod of the present invention, further comprises 0 to 10 wt% of an optional additive. More preferably, the free standing film used in the personal care pod of the present invention, further comprises 0 to 10 wt% of an optional additive; wherein the optional additive is selected from the group consisting of a preservative, an antioxidant, a viscosity modifier, a solubility modifier, an antimicrobial agent, a binder, a chelating, a filler, an extender, a defoamer, a lubricant, a release agent, an anti-blocking agent, a tackifier, a coalescent, a detackifying agent and a nanoparticle (e.g., silicate type nanoclay).

Preferably, the free standing film used in the personal care pod of the present invention, further comprises 0 to 10 wt% of an optional additive, wherein the optional additive includes a nanoparticle (preferably, a silicate type nanoclay). More preferably, the free standing film used in the personal care pod of the present invention, further comprises 0.05 to 1 wt% (more preferably, 0.1 to 0.5 wt%; most preferably, 0.1 to 0.3 wt%) of a nanoparticle (preferably, a silicate type nanoclay). Most preferably, the free standing film used in the personal care pod of the present invention, further comprises 0.1 to 0.3 wt% of a nanoparticle (preferably, a silicate type nanoclay).

Preferably, the free standing film used in the personal care pod of the present invention, further comprises 0 to 10 wt% of an optional additive, wherein the optional additive includes a defoamer. More preferably, the free standing film used in the personal care pod of the present invention, further comprises 1 to 10 wt% (more preferably, 2 to 7.5 wt%; most preferably, 3 to 6 wt%) of an defoamer. Most preferably, the free standing film used in the personal care pod of the present invention, further comprises 3 to 6 wt% of a defoamer.

the free standing film used in the personal care pod of the present invention can be prepared by techniques known to those skilled in the art including, for example, via solution casting on a substrate, such as glass, polyethylene terephthalate (PET) or metal. Typically, water is used as the solvent for the solution casting, although other solvents may be used. Following casting, the films may be dried by heating at elevated temperature, for instance 65-80 °C.

Preferably, the personal care formulation included in the personal care pod of the present invention is selected from the group consisting of a shampoo, a conditioning shampoo, a hair conditioner, a hand cleanser, a facial wash, a body wash, a skin moisturizing formulation (hand/face/body), a shaving cream and a topical medicament. More preferably, the personal care formulation included in the personal care pod of the present invention is selected from the group consisting of a hand cleanser, a facial wash, a skin moisturizing formulation (hand/face/body) and a shaving cream. Still more preferably, the personal care formulation included in the personal care pod of the present invention is selected from the group consisting of a hand cleanser, a facial wash and a shaving cream. Most preferably, the personal care formulation included in the personal care pod of the present invention is a hand cleanser.

Preferably, the personal care pod of the present invention is a single use personal care pod, wherein the personal care pod is formulated to be exposed to water by a user and then used (e.g., to wash the user's hands, face or hair).

Preferably, the personal care formulation included in the personal care pod of the present invention is a personal care concentrate-i.e., wherein the personal care formulation incorporated into the personal care pod is formulated for dilution in a sufficient volume of water to facilitate formation of a large volume of personal care formulation for use by the end user (e.g., consumer). Preferably, wherein the large volume is enough to enable at least two applications; preferably, at least five applications; more preferably, at least 10 applications of the personal care formulation (e.g., at least two separate hair washings for a shampoo, at least two hand washings for a hand cleanser).

Preferably, the personal care formulation included in the personal care pod of the present invention comprises at least one of dermatologically acceptable cleaning surfactant and a conditioning agent.

Preferably, the personal care formulation included in the personal care pod of the present invention comprises 0 to 20 wt% (preferably, 0 to 15 wt%; more preferably, 0 to 10 wt%; most preferably, 0 to 7.5 wt%), based on weight of the personal care formulation, of water.

Preferably, the personal care formulation included in the personal care pod of the present invention includes a dermatologically acceptable cleaning surfactant; wherein the dermatologically acceptable cleaning surfactant is selected from the group consisting of alkyl polyglucosides (e.g., lauryl glucoside, coco-glucoside, decyl glucoside), glycinates (e.g., sodium cocoyl glycinate), betaines (e.g., alkyl betaines such as cetyl betaine and amido betaines such as cocamidopropyl betaine), taurates (e.g., sodium methyl cocoyl taurate), glutamates (e.g., sodium cocoyl glutamate), sarcosinates (e.g., sodium lauroyl sarcosinate), isethionates (e.g., sodium cocoyl isethionate, sodium lauroyl methyl isethionate), sulfoacetates (e.g., sodium lauryl sulfoacetate), alaninates (e.g., sodium cocoyl alaninate), amphoacetates (e.g., sodium cocoamphoacetate), sulfates (e.g., sodium lauryl ether sulfate (SLES)), sulfonates (e.g., sodium C₁₄₋₁₆ olefin sulfonate), succinates (e.g., disodium lauryl sulfosuccinate), fatty alkanolamides (e.g., cocamide monoethanolamine, cocamide diethanolamine, soyamide diethanolamine, lauramide diethanolamine, oleamide monoisopropanolamine, stearamide monoethanolamine, myristamide monoethanolamine, lauramide monoethanolamine, capramide diethanolamine, ricinoleamide diethanolamine, myristamide diethanolamine, stearamide diethanolamine, oleylamide diethanolamine, tallowamide diethanolamine, lauramide monoisopropanolamine, tallowamide monoethanolamine, isostearamide diethanolamine, isostearamide monoethanolamine) and mixtures thereof. More preferably, the personal care formulation included in the personal care pod of the present invention includes a dermatologically acceptable cleaning surfactant; wherein the dermatologically acceptable cleaning surfactant includes a sodium lauryl ether sulfate. Most preferably, the personal care formulation included in the personal care pod of the present invention includes a dermatologically acceptable cleaning surfactant; wherein the dermatologically acceptable cleaning surfactant includes a blend of a sodium lauryl ether sulfate, a cocamide monoethanolamine and a cocamidopropyl betaine.

Preferably, the personal care formulation included in the personal care pod of the present invention comprises a dermatologically acceptable cleaning surfactant

Preferably, the personal care formulation included in the personal care pod of the present invention comprises 5 to 85 wt% (preferably, 10 to 75 wt%; more preferably, 20 to 60 wt%; most preferably, 25 to 40 wt%), based on weight of the personal care formulation, of a dermatologically acceptable cleaning surfactant.

Preferably, the dermatologically acceptable cleaning surfactant is in isotropic phase in the personal care formulation included in the personal care pod of the present invention. The term "isotropic phase" as used herein and in the appended claims in reference to the dermatologically acceptable cleaning surfactant means that the individual molecules of the surfactant are associated, but remain in the micellar state. The micelles can have a spherical shape or may be elongated at higher concentrations, but maintain mobility and dynamic exchange and dissolve readily. This is in contrast to the gel phase where elongated micelles begin to pack in arrays, e.g., long cylindrical structures and have lost their mobility and dynamic exchange capability and therefore do not dissolve or disperse readily upon dilution.

Preferably, the personal care formulation included in the personal care pod of the present invention includes a dermatologically acceptable silicone; wherein the dermatologically acceptable silicone is selected from the group consisting of amodimethicone, cyclomethicone, dimethicone, dimethiconol, hexadecyl methicone, hexamethyldisiloxane, diisopropanol amino-PG-propyl disiloxane, methicone, phenyl dimethicone, bis-vinyl dimethicone, stearoxy dimethicone polyalkyl siloxane, polyalkylaryl siloxane, silicone gums (i.e., polydiorganosiloxanes having a weight average molecular weight of 200,000 to 1,000,000 Daltons), polyaminofunctional silicones (e.g., DOWSIL^{™} 969 Emulsion, DOWSIL^{™} CE-7081 Smart Style, DOWSIL^{™} 5-7113 Silicone Quat MicroEmulsion) and combinations thereof.

Preferably, the personal care formulation included in the personal care pod of the present invention comprises 1 to 20 wt% (preferably, 2 to 15 wt%; more preferably, 2.5 to 10 wt%; most preferably, 4 to 8 wt%), based on weight of the personal care formulation, of a dermatologically acceptable silicone.

Preferably, the personal care formulation included in the personal care pod of the present invention optionally further comprises at least one additional ingredient selected from the group consisting of an antimicrobial agent/preservative (e.g., benzoic acid, sorbic acid, phenoxyethanol, methylisothiazolinone, ethylhexyl glycerin); a rheology modifier (e.g., PEG-150 pentaerythrityl tetrastearate); a soap; a colorant; pH adjusting agent; an antioxidant (e.g., butylated hydroxytoluene); a humectant (e.g., glycerin, sorbitol, monoglycerides, lecithins, glycolipids, fatty alcohols, fatty acids, polysaccharides, sorbitan esters, polysorbates (e.g., Polysorbate 20, Polysorbate 40, Polysorbate 60, and Polysorbate 80), diols (e.g., propylene glycol), diol analogs, triols, triol analogs, cationic polymeric polyols); a wax; a foaming agent; an emulsifying agent; a fragrance; a chelating agent (e.g., tetrasodium ethylene diamine tetraacetic acid); a preservative (e.g., benzoic acid, sorbic acid, phenoxyethanol, methylisothiazolinone); a bleaching agent; a lubricating agent; a sensory modifier; a sunscreen additive; a vitamin; a protein/amino acid; a plant extract; a natural ingredient; a bioactive agent; an anti-aging agent; a pigment; an acid; a penetrant; an anti-static agent; an anti-frizz agent; an antidandruff agent; a hair waving/straightening agent; a hair styling agent; a hair oil; natural oils or ester emollients (e.g., mono-, di-, tri-glycerides such as sunflower seed oil, coconut oil, cottonseed oil, borage oil, borage seed oil, primrose oil, castor and hydrogenated castor oils, rice bran oil, soybean oil, olive oil, safflower oil, shea butter, jojoba oil and combinations thereof); an absorbent; a hard particle; a soft particle; a conditioning agent (e.g., guar hydroxypropyltrimonium chloride, PQ-10, PQ-7); a slip agent; an opacifier; a pearlizing agent and a salt.

Preferably, the method of using a personal care pod of the present invention, wherein the personal care pod is a single use personal care pod, comprises: applying water to the single use personal care pod to dissolve or disperse the free standing film and release the personal care formulation; and applying the personal care formulation to skin or hair (preferably, mammalian skin or hair; more preferably, human skin or hair). Preferably, the personal care formulation is applied to the skin or hair concomitantly with the dissolution or dispersal of the free standing film. More preferably, wherein the personal care formulation is applied to the skin or hair simultaneously with the dissolution or dispersal of the free standing film.

Preferably, the method of using a personal care pod of the present invention, wherein the personal care pod is a personal care concentrate pod and wherein the personal care formulation contained in the personal care concentrate pod is a personal care formulation concentrate: providing a container (preferably, wherein the contain has a water level marking to facilitate accurate dosing); adding water to the container; adding the personal care concentrate pod to the container; agitating the container contents; allowing the water to dissolve or disperse the free standing film of the personal care concentrate pod; and mixing the water with the personal care formulation concentrate from the personal care concentrate pod to form a use concentration personal care formulation in the container. The term "use concentration" as used herein and in the appended claims in reference to a personal care formulation means the concentration used when applying the formulation to the skin or hair.

Some embodiments of the present invention will now be described in detail in the following **Examples.**

### Synthesis S1: Crosslinked Cellulose Ether

The crosslinking agent used in **Synthesis S1** was a linear poly(propyleneglycol) diglycidyl ether made from polypropylene glycol (PPG) having a molecular weight of ~400 Daltons and having the formula 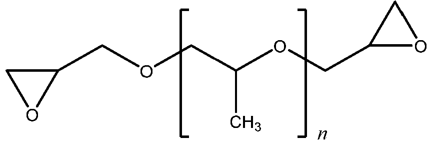 wherein n is 5.7 to 6.7 (available from Leuna-Harze GmbH, Leuna, DE as EPILOX^{™} M985 poly(propyleneglycol) diglycidylether crosslinker).

Ground cellulose flock (1.5 mol) was added to a 5 L autoclave. After purging the autoclave trice with nitrogen gas, the contents of the autoclave were heated to 40 °C. Then dimethylether (DME, 4.7 mol/mol of anhydroglucose units (AGU)) and methyl chloride (MCl; 3.2 mol/mol AGU) were injected into the autoclave. Caustic soda (NaOH, strength 50 wt% aqueous, 1.9 mol NaOH/mol AGU) was added to the autoclave in 3 portions during 2 minutes at a temperature of 40 °C. The reaction mixture was held at 40 °C for 30 minutes. Ethylene oxide (0.45 mol/mol AGU) was then added and the reaction mixture was held for 10 minutes at 40 °C. The crosslinker (EPILOX^{™} M985 crosslinker; 0.0025 mol/mol AGU) was dissolved in 20 mL of isopropanol and added to the contents of the autoclave in six increments in 30 second intervals. The contents of the autoclave were then heated to 80 °C in 40 minutes. At 80 °C a water soluble monovalent copper ligand (MCL 2; 1.3 mol/mol AGU) was injected into the autoclave quickly. Afterwards, NaOH (0.67 mol/mol AGU) was added in 7 portions over 30 minutes, followed by a 70 minute cook-off time at 80 °C. Following this, the product crosslinked cellulose ether was washed in hot (> 95 °C) water, neutralized with formic acid, granulated, dried and milled.

### Comparative Examples C1-C18 and Examples 1-15: Film/Additive Blends

Water soluble film/additive blends were prepared according to **Comparative Examples C1-C18** and **Examples 1-15** by mixing together the components in the concentrations noted in TABLE 1.

**TABLE 1**

| **Example** | **Ingredient (wt%)** | | | | | |
|---|---|---|---|---|---|---|
| | **Water soluble film** | | | **Additive** | | **Water** |
| | **A** | **B** | **C** | **Description** | **(wt%)** | |
| **C1** | 1 | -- | -- | - - | -- | 99 |
| **C2** | - - | 1 | -- | - - | -- | 99 |
| **C3** | - - | -- | 1 | - - | -- | 99 |
| **C4** | - - | -- | 0.9 | D | 0.1 | 99 |
| **C5** | - - | -- | 0.75 | D | 0.25 | 99 |
| **C6** | - - | -- | 0.5 | D | 0.5 | 99 |
| **C7** | - - | -- | 0.25 | D | 0.75 | 99 |
| **C8** | - - | -- | 0.1 | D | 0.9 | 99 |
| **C9** | - - | -- | 0.9 | E | 0.1 | 99 |
| **C10** | - - | -- | 0.75 | E | 0.25 | 99 |
| **C11** | - - | -- | 0.5 | E | 0.5 | 99 |
| **C12** | - - | -- | 0.25 | E | 0.75 | 99 |
| **C13** | - - | -- | 0.1 | E | 0.9 | 99 |
| **C14** | - - | -- | 0.9 | F | 0.1 | 99 |
| **C15** | - - | -- | 0.75 | F | 0.25 | 99 |
| **C16** | - - | -- | 0.5 | F | 0.5 | 99 |
| **C17** | - - | -- | 0.25 | F | 0.75 | 99 |
| **C18** | - - | -- | 0.1 | F | 0.9 | 99 |
| **1** | 0.9 | -- | - - | G | 0.1 | 99 |
| **2** | 0.75 | -- | - - | G | 0.25 | 99 |
| **3** | 0.5 | -- | - - | G | 0.5 | 99 |
| **4** | 0.25 | -- | - - | G | 0.75 | 99 |
| **5** | 0.1 | -- | - - | G | 0.9 | 99 |
| **6** | - - | 0.9 | - - | G | 0.1 | 99 |
| **7** | - - | 0.75 | - - | G | 0.25 | 99 |
| **8** | - - | 0.5 | - - | G | 0.5 | 99 |
| **9** | - - | 0.25 | - - | G | 0.75 | 99 |
| **10** | - - | 0.1 | - - | G | 0.9 | 99 |
| **11** | - - | -- | 0.9 | G | 0.1 | 99 |
| **12** | - - | -- | 0.75 | G | 0.25 | 99 |
| **13** | - - | -- | 0.5 | G | 0.5 | 99 |
| **14** | - - | -- | 0.25 | G | 0.75 | 99 |
| **15** | - - | -- | 0.1 | G | 0.9 | 99 |
| ^{A} MonoSol M8312 polyvinyl alcohol based film from MonoSol LLC | | | | | | |
| ^{B} Solublon GA-BTX 75 µm polyvinyl alcohol based film from Aicello Corporation | | | | | | |
| ^{C} Solublon GA-BTX 90 µm polyvinyl alcohol based film from Aicello Corporation | | | | | | |
| ^{D} SUPRACARE^{™} 760 additive from The Dow Chemical Company | | | | | | |
| ^{E} CELLOSIZE^{™} QP 100 MH from The Dow Chemical Company | | | | | | |
| ^{F} CELLOSIZE^{™} Texture 40-0202 from The Dow Chemical Company | | | | | | |
| ^{G} Product of **Synthesis S1** | | | | | | |

### Viscosity measures of film blended with additive

The viscosity of the water soluble film/additive blends prepared according to **Comparative Examples C1-C18** and **Examples 1-15** were measured using a Brookfield DV-II programmable viscometer with a CP-52 spindle at 25 °C. Measurements were taken at 5 rpm, 10 rpm, 20 rpm and 50 rpm for each blend and are reported in **TABLE 2.**

**TABLE 2**

| **Example** | **Viscosity (cP)** | | | |
|---|---|---|---|---|
| | **5 rpm** | **10 rpm** | **20 rmp** | **50 rpm** |
| **C1** | 1 | 1 | 1 | 1 |
| **C2** | 1 | 1 | 1 | 1 |
| **C3** | 1 | 1 | 1 | 1 |
| **C4** | 1 | 1 | 1 | 1 |
| **C5** | 1 | 20 | 20 | 20 |
| **C6** | 238 | 260 | 200 | 130 |
| **C7** | 1,110 | 800 | 580 | 350 |
| **C8** | 2,000 | 1,380 | 930 | 530 |
| **C9** | 40 | 40 | 5 | 16 |
| **C10** | 60 | 40 | 40 | 32 |
| **C11** | 280 | 250 | 170 | 120 |
| **C12** | 890 | 650 | 470 | 280 |
| **C13** | 1,630 | 1,120 | 750 | 430 |
| **C14** | 40 | 30 | 5 | 10 |
| **C15** | 140 | 70 | 20 | 15 |
| **C16** | 100 | 70 | 20 | 50 |
| **C17** | 180 | 130 | 100 | 80 |
| **C18** | 240 | 190 | 150 | 120 |
| **1** | 1 | 1 | 5 | 6 |
| **2** | 40 | 40 | 40 | 32 |
| **3** | 540 | 370 | 260 | 160 |
| **4** | 1,700 | 1,130 | 740 | 410 |
| **5** | 2,860 | 1,830 | 1,170 | 620 |
| **6** | 1 | 1 | 1 | 2 |
| **7** | 60 | 40 | 30 | 26 |
| **8** | 220 | 210 | 160 | 105 |
| **9** | 730 | 580 | 450 | 290 |
| **10** | 1,530 | 1,040 | 810 | 500 |
| **11** | 1 | 1 | 5 | 6 |
| **12** | 60 | 50 | 45 | 35 |
| **13** | 400 | 310 | 230 | 150 |
| **14** | 1,230 | 870 | 610 | 370 |
| **15** | 2,460 | 1,700 | 1,130 | 640 |

### Examples 16-25: Water soluble film

Water soluble films were formed in each of **Examples 16-25** by placing a water soluble film/additive blend prepared according to one of **Examples 1-10,** as noted in **TABLE 3,** in a vented oven at 60 °C and drying to form the film. Once formed, the films were then redispersed in water forming a 1 wt% solution in water. The viscosity of these 1 wt% solutions were then measured using a Brookfield DV-II programmable viscometer with a CP-52 spindle at 25 °C. Measurements were taken at 5 rpm, 10 rpm, 20 rpm and 50 rpm for each blend and are reported in **TABLE 3.**

**TABLE 3**

| **Example** | **Starting Blend** | **Viscosity (cP)** | | | |
|---|---|---|---|---|---|
| | | **5 rpm** | **10 rpm** | **20 rmp** | **50 rpm** |
| **16** | **Example 1** | 1 | 1 | 1 | 2 |
| **17** | **Example 2** | 120 | 120 | 100 | 75 |
| **18** | **Example 3** | 400 | 320 | 240 | 150 |
| **19** | **Example 4** | 3,400 | 2,230 | 1,420 | 760 |
| **20** | **Example 5** | 4,500 | 2,900 | 1,840 | 950 |
| **21** | **Example 6** | 1 | 1 | 1 | 1 |
| **22** | **Example 7** | 60 | 40 | 30 | 20 |
| **23** | **Example 8** | 200 | 150 | 105 | 80 |
| **24** | **Example 9** | 520 | 400 | 340 | 220 |
| **25** | **Example 10** | 900 | 610 | 500 | 340 |

### Prophetic Formulation F1: Concentrated shampoo formulation

Shampoo formulation of **Prophetic Formulation F1** is prepared by mixing together the components in the weight proportions noted in **TABLE 4.**

**TABLE 4**

| **Ingredient INCI name** | **F1** |
|---|---|
| | **wt%** |
| Decyl glucoside | 19 |
| Disodium laureth sulfosuccinate | 24 |
| Cocamidopropyl betaine | 39 |
| Sodium lauroyl sarcosinate | 12 |
| Phenoxyethanol | 1.2 |
| Deionized water | 4.8 |

### Propheric Film resistance to concentrated shampoo formulation

A drop of the prophetic concentrated shampoo formulation according to **Prophetic Formulation F2** is applied to the surface of a water soluble films formed according to each of **Examples 16-25.** Each of the films are observed to resist penetration of the concentrated hard surface cleaning composition.

### Synthesis S2: Free standing film

Crosslinked cellulose ether prepared according to **Synthesis S1** (1 g), calcium chloride dihydrate (0.4 g) and deionized water (98.6 g) were mixed together. The mixture was then deposited on a substrate and dried in a vented oven at 60 °C to form a film.

### Free standing Film resistance to concentrated shampoo formulation

Prophetic concentrated shampoo formulation according to **Prophetic Formulation F1** is added to an Ependorf tube. The free standing film prepared according to **Synthesis S2** is used to cap the opening of the Ependorf tube. The Ependorf tube is then turned over with the opening facing down so that the concentrated shampoo formulation inside the tube is supported by the free standing film. The free standing film is observed to resist the penetration of the concentrated shampoo formulation.

## Claims

1. A personal care pod, comprising:
a free standing film, wherein the free standing film comprises 20 to 100 wt%, based on weight of the free standing film, of a crosslinked cellulose ether containing 0.1 to 0.6 wt%, based on weight of the crosslinked cellulose ether, of polyether groups; and
a personal care formulation;
wherein the free standing film forms a cavity; wherein the personal care formulation is disposed within the cavity; wherein the personal care formulation is in contact with the free standing film; and wherein the free standing film encapsulates the personal care formulation.

2. The personal care pod of claim 1, wherein the personal care formulation is selected from the group consisting of a shampoo, a conditioning shampoo, a hair conditioner, a hand cleanser, a facial wash, a body wash, a skin moisturizing formulation (hand/face/body), a shaving cream and a topical medicament.

3. The personal care pod of claim 1, wherein the free standing film has an average thickness of 5 to 200 µm.

4. The personal care pod of claim 3, wherein the crosslinked cellulose ether is an irreversibly crosslinked cellulose ether; and wherein the polyether groups in the irreversibly crosslinked cellulose ether are polyoxyalkylene groups having 2 to 100 oxyalkylene groups.

5. The personal care pod of claim 4, wherein the irreversibly crosslinked cellulose ether comprises a base cellulose ether and crosslinks; wherein the crosslinks contain the polyether groups; and wherein the base cellulose ether contains hydroxyalkyl ether and alkyl ether groups.

6. The personal care pod of claim 1, further comprising at least one of:
(a) 5 to 80 wt%, based on weight of the free standing film, of a partially hydrolyzed poly(vinyl acetate);
(b) 0.1 to 45 wt%, based on weight of the free standing film, of a crosslinking agent;
(c) 0.1 to 45 wt%, based on weight of the free standing film, of a poly(ethylene oxide) having a weight average molecular weight of 20,000 to 2,000,000 Daltons;
(d) 0.1 to 35 wt%, based on weight of the free standing film, of a poly(alkylene glycol) having a weight average molecular weight of > 400 to 5,300 Daltons;
(e) 0.1 to 45 wt%, based on weight of the free standing film, of a plasticizer;
(f) 0.1 to 10 wt%, based on weight of the free standing film, of a poly(isobutylene-co-maleic anhydride) copolymer, wherein the poly(isobutylene-co-maleic anhydride) copolymer is at least partially neutralized; and
(g) 0.01 to 1.8 wt%, based on weight of the free standing film, of a poly(vinyl pyrrolidone).

7. The personal care pod of claim 1, wherein the free standing film comprises:
20 to 95 wt%, based on weight of the free standing film, of the crosslinked cellulose ether containing 0.1 to 0.6 wt%, based on weight of the crosslinked cellulose ether, of polyether groups; and
5 to 80 wt%, based on weight of the free standing film, of the partially hydrolyzed poly(vinyl acetate).

8. The personal care pod of claim 7, wherein the free standing film further comprising an optional additive; wherein the optional additive is selected from the group consisting of a preservative, an antioxidant, a viscosity modifier, a solubility modifier, an antimicrobial agent, a binder, a chelating agent, a filler, an extender, a defoamer, a lubricant, a release agent, an anti-blocking agent, a tackifier, a coalescent, a detackifying agent and a nanoparticle.

9. A non-therapeutic method of using a personal care pod of claim 1, wherein the personal care pod is a single use personal care pod, comprising:
applying water to the single use personal care pod to dissolve or disperse the free standing film and release the personal care formulation; and
applying the personal care formulation to skin or hair.

10. A method of forming a personal care formulation, comprising:
providing a personal care pod of claim 1; wherein the personal care pod is a personal care concentrate pod and wherein the personal care formulation contained in the personal care concentrate pod is a personal care formulation concentrate;
providing a container;
adding water to the container;
adding the personal care concentrate pod to the container;
agitating the container contents;
allowing the water to dissolve or disperse the free standing film of the personal care concentrate pod; and
mixing the water with the personal care formulation concentrate from the personal care concentrate pod to form a use concentration personal care formulation in the container.

## Patentansprüche

1. Körperpflegekapsel, umfassend:
einen freistehenden Film, wobei der freistehende Film, bezogen auf das Gewicht des freistehenden Films, 20 bis 100 Gew.-% eines vernetzten Celluloseethers umfasst, der, bezogen auf das Gewicht des vernetzten Celluloseethers, 0,1 bis 0,6 Gew.-% an Polyethergruppen enthält; und
eine Körperpflegezusammensetzung;
wobei der freistehende Film einen Hohlraum bildet; wobei die Körperpflegezusammensetzung in dem Hohlraum angeordnet ist; wobei die Körperpflegezusammensetzung mit dem freistehenden Film in Kontakt steht; und wobei der freistehende Film die Körperpflegezusammensetzung einkapselt.

2. Körperpflegekapsel nach Anspruch 1, wobei die Körperpflegezusammensetzung ausgewählt ist aus der Gruppe bestehend aus einem Shampoo, einem Pflegeshampoo, einer Haarspülung, einem Handreiniger, einem Gesichtswaschmittel, einem Körperwaschmittel, einer Hautbefeuchtungszusammensetzung (Hand/Gesicht/Körper), einer Rasiercreme und einem topischen Arzneimittel.

3. Körperpflegekapsel nach Anspruch 1, wobei der freistehende Film eine durchschnittliche Dicke von 5 bis 200 µm aufweist.

4. Körperpflegekapsel nach Anspruch 3, wobei der vernetzte Celluloseether ein irreversibel vernetzter Celluloseether ist; und wobei die Polyethergruppen in dem irreversibel vernetzten Celluloseether Polyoxyalkylengruppen sind, die 2 bis 100 Oxyalkylengruppen aufweisen.

5. Körperpflegekapsel nach Anspruch 4, wobei der irreversibel vernetzte Celluloseether einen Basiscelluloseether und Vernetzungen umfasst; wobei die Vernetzungen die Polyethergruppen enthalten; und wobei der Basiscelluloseether Hydroxyalkylether- und Alkylethergruppen enthält.

6. Körperpflegekapsel nach Anspruch 1, ferner umfassend mindestens eines von:
(a) 5 bis 80 Gew.-% eines teilweise hydrolysierten Poly(vinylacetats) bezogen auf das Gewicht des freistehenden Films;
(b) 0,1 bis 45 Gew.-% eines Vernetzungsmittels bezogen auf das Gewicht des freistehenden Films;
(c) 0,1 bis 45 Gew.-% eines Poly(ethylenoxids) mit einem Gewichtsmittel des Molekulargewichts von 20.000 bis 2.000.000 Dalton bezogen auf das Gewicht des freistehenden Films;
(d) 0,1 bis 35 Gew.-% eines Poly(alkylenglykols) mit einem Gewichtsmittel des Molekulargewichts von > 400 bis 5.300 Dalton bezogen auf das Gewicht des freistehenden Films;
(e) 0,1 bis 45 Gew.-% eines Weichmachers bezogen auf das Gewicht des freistehenden Films;
(f) 0,1 bis 10 Gew.-% eines Poly(isobutylen-co-maleinsäureanhydrid)-Copolymers bezogen auf das Gewicht des freistehenden Films, wobei das Poly(isobutylen-co-maleinsäureanhydrid)-Copolymer zumindest teilweise neutralisiert ist; und
(g) 0,01 bis 1,8 Gew.-% eines Poly(vinylpyrrolidons) bezogen auf das Gewicht des freistehenden Films.

7. Körperpflegekapsel nach Anspruch 1, wobei der freistehende Film Folgendes umfasst:
bezogen auf das Gewicht des freistehenden Films 20 bis 95 Gew.-% des vernetzten Celluloseethers, der 0,1 bis 0,6 Gew.-% an Polyethergruppen bezogen auf das Gewicht des vernetzten Celluloseethers enthält; und
bezogen auf das Gewicht des freistehenden Films 5 bis 80 Gew.-% des teilweise hydrolysierten Poly(vinylacetats).

8. Körperpflegekapsel nach Anspruch 7, wobei der freistehende Film ferner ein optionales Additiv umfasst; wobei das optionale Additiv ausgewählt ist aus der Gruppe bestehend aus einem Konservierungsmittel, einem Antioxidans, einem Viskositätsmodifikator, einem Löslichkeitsmodifikator, einem antimikrobiellen Mittel, einem Bindemittel, einem Chelatbildner, einem Füllstoff, einem Streckmittel, einem Entschäumer, einem Schmiermittel, einem Trennmittel, einem Antiblockiermittel, einem Klebrigmacher, einem Koalesziermittel, einem Entklebungsmittel und einem Nanoteilchen.

9. Nicht-therapeutisches Verfahren zum Verwenden einer Körperpflegekapsel nach Anspruch 1, wobei die Körperpflegekapsel eine Einweg-Körperpflegekapsel ist, umfassend:
das Aufbringen von Wasser auf die Einweg-Körperpflegekapsel, um den freistehenden Film aufzulösen oder zu dispergieren und die Körperpflegezusammensetzung freizusetzen; und
das Auftragen der Körperpflegezusammensetzung auf die Haut oder das Haar.

10. Verfahren zum Herstellen einer Körperpflegezusammensetzung, umfassend:
Bereitstellen einer Körperpflegekapsel nach Anspruch 1; wobei die Körperpflegekapsel eine Körperpflegekonzentratkapsel ist und wobei die in der Körperpflegekonzentratkapsel enthaltene Körperpflegezusammensetzung ein Körperpflegezusammensetzungskonzentrat ist;
Bereitstellen eines Behälters;
Zugabe von Wasser in den Behälter;
Zugabe der Körperpflegekonzentratkapsel in den Behälter;
Rühren des Behälterinhalts;
Zulassen, dass das Wasser den freistehenden Film der Körperpflegekonzentratkapsel auflöst oder dispergiert; und
Mischen des Wassers mit dem Körperpflegezusammensetzungskonzentrat aus der Körperpflegekonzentratkapsel zum Ausbilden einer Körperpflegezusammensetzung in Gebrauchskonzentration in dem Behälter.

## Revendications

1. Dosette de soins personnels, comprenant :
un film autonome, dans laquelle le film autonome comprend 20 à 100 % en poids, sur la base du poids du film autonome, d'un éther de cellulose réticulé contenant 0,1 à 0,6 % en poids, sur la base du poids de l'éther de cellulose réticulé, de groupes polyéthers ; et
une formulation de soins personnels ;
dans laquelle le film autoportant forme une cavité ; dans laquelle la formulation de soins personnels est disposée au sein de la cavité ; dans laquelle la formulation de soins personnels est en contact avec le film autonome ; et dans laquelle le film autonome encapsule la formulation de soins personnels.

2. Dosette de soins personnels selon la revendication 1, dans laquelle la formulation de soins personnels est choisie dans le groupe constitué d'un shampooing, d'un shampooing revitalisant, d'un revitalisant capillaire, d'un nettoyant pour les mains, d'un nettoyant pour le visage, d'un nettoyant pour le corps, d'une formulation hydratante pour la peau (mains/visage/corps), d'une crème à raser et d'un médicament topique.

3. Dosette de soins personnels selon la revendication 1, dans laquelle le film autonome a une épaisseur moyenne de 5 à 200 µm.

4. Dosette de soins personnels selon la revendication 3, dans laquelle l'éther de cellulose réticulé est un éther de cellulose irréversiblement réticulé ; et dans laquelle les groupes polyéther dans l'éther de cellulose irréversiblement réticulé sont des groupes polyoxyalkylène ayant de 2 à 100 groupes oxyalkylène.

5. Dosette de soins personnels selon la revendication 4, dans laquelle l'éther de cellulose irréversiblement réticulé comprend un éther de cellulose de base et des réticulations ; dans laquelle les réticulations contiennent les groupes polyéther ; et dans laquelle l'éther de cellulose de base contient des groupes hydroxyalkyléther et alkyléther.

6. Dosette de soins personnels selon la revendication 1, comprenant en outre au moins l'un parmi :
(a) 5 à 80 % en poids, sur la base du poids du film autonome, d'un poly(acétate de vinyle) partiellement hydrolysé ;
(b) 0,1 à 45 % en poids, sur la base du poids du film autonome, d'un agent de réticulation ;
(c) 0,1 à 45 % en poids, sur la base du poids du film autonome, d'un poly(oxyde d'éthylène) ayant une masse moléculaire moyenne en poids de 20 000 à 2 000 000 Daltons ;
(d) 0,1 à 35 % en poids, sur la base du poids du film autonome, d'un poly(alkylène glycol) ayant une masse moléculaire moyenne en poids de > 400 à 5 300 Daltons ;
(e) 0,1 à 45 % en poids, sur la base du poids du film autonome, d'un plastifiant ;
(f) 0,1 à 10 % en poids, sur la base du poids du film autonome, d'un copolymère poly(isobutylène-co-anhydride maléique), dans laquelle le copolymère poly(isobutylène-co-anhydride maléique) est au moins partiellement neutralisé ; et
(g) 0,01 à 1,8 % en poids, sur la base du poids du film autonome, d'une poly(vinylpyrrolidone).

7. Dosette de soins personnels selon la revendication 1, dans laquelle le film autoportant comprend :
20 à 95 % en poids, sur la base du poids du film autonome, de l'éther de cellulose réticulé contenant 0,1 à 0,6 % en poids, sur la base du poids de l'éther de cellulose réticulé, de groupes polyéther ; et
5 à 80 % en poids, sur la base du poids du film autonome, du poly(acétate de vinyle) partiellement hydrolysé.

8. Dosette de soins personnels selon la revendication 7, dans laquelle le film autonome comprend en outre un additif facultatif ; dans laquelle l'additif facultatif est choisi dans le groupe constitué d'un conservateur, d'un antioxydant, d'un modificateur de viscosité, d'un modificateur de solubilité, d'un agent antimicrobien, d'un liant, d'un agent chélateur, d'une charge, d'un diluant, d'un antimousse, d'un lubrifiant, d'un agent de démoulage, d'un agent anti-blocage, d'un agent collant, d'un agent de coalescence, d'un agent de suppression d'adhésivité et d'une nanoparticule.

9. Procédé non thérapeutique d'utilisation d'une dosette de soins personnels selon la revendication 1, dans lequel la dosette de soins personnels est une dosette de soins personnels à usage unique, comprenant :
l'application d'eau à la dosette de soins personnels à usage unique afin de dissoudre ou de disperser le film autonome et de libérer la formulation de soins personnels ; et
l'application de la formulation de soins personnels sur la peau ou les cheveux.

10. Procédé de formation d'une formulation de soins personnels, comprenant :
la fourniture d'une dosette de soins personnels selon la revendication 1 ; dans lequel la dosette de soins personnels est une dosette de concentré de soins personnels et dans lequel la formulation de soins personnels contenue dans la dosette de concentré de soins personnels est un concentré de formulation de soins personnels ;
la fourniture d'un récipient ;
l'ajout d'eau dans le récipient ;
l'ajout de la dosette de concentré de soins personnels dans le récipient ;
l'agitation du contenu du récipient ;
le fait de laisser l'eau dissoudre ou disperser le film autonome de la dosette de concentré de soins personnels ; et
le mélange de l'eau avec le concentré de formulation de soins personnels provenant de la dosette de concentré de soins personnels afin de former une formulation de soins personnels à concentration d'utilisation dans le récipient.
